# EUROPEAN PATENT APPLICATION

(11) **EP 3 524 589 A1**
(43) Date of publication of application: **14.08.2019**
(21) Application number: 18155929.5
(22) Date of filing: 09.02.2018
(51) Int. Cl.: C07C 51/56, C07C 51/60

(54) **PROCESS FOR THE MANUFACTURING OF A (METH)ACRYLIC ANHYDRIDE IN A FLOW REACTOR**

(71) Applicant: 3M Innovative Properties Company, Saint Paul, MN 55133-3427 (US)
(72) Inventor: Dams, Rudolf, 2070 Zwijndrecht (BE); Van Campenhout, Rudy, 2070 Zwijndrecht (BE); Conradi, Matthias, 2070 Zwijndrecht (BE); Movsisyan, Marine, 9000 Gent (BE); Stevens, Christian, 9000 Gent (BE)
(74) Representative: Gabriel, Kiroubagaranne

(57) **Abstract**

The present disclosure relates to a process for the manufacturing of a (meth)acrylic anhydride, wherein the process comprises the steps of:
A. providing a flow reactor comprising a reaction chamber;
B. providing reactants and reagents comprising:
a) a (meth)acryloyl halide;
b) an organic solvent;
c) (meth)acrylic acid;
d) and either:
i. a tertiary amine; or
ii. an inorganic base and a polar solvent; and

C. incorporating the reactants and reagents into the reaction chamber of the flow reactor, thereby forming a reaction product stream comprising the (meth)acrylic anhydride.

In another aspect, the present disclosure is directed to the use of a polar solvent for the manufacturing of a (meth)acrylic anhydride in a flow reactor.

## Description

### Technical Field

The present disclosure relates to the field of manufacturing (meth)acrylic anhydrides in flow reactors.

### Background

Acid anhydrides, in particular (meth)acrylic anhydrides, can produce valuable synthetic intermediates. The broad utility of acid anhydrides, in particular (meth)acrylic anhydrides, has drawn tremendous attention over the years. This class of compounds is important to facilitate numerous synthetic transformations owing to their high reactivity. Among this category of compounds, alpha,beta-unsaturated acid anhydrides, and in particular (meth)acrylic anhydrides, have received significant attention since the late 20^{th} century. These specific acid anhydrides are highly reactive intermediates which can be used for the production of important (meth)acrylates and polymers with commercial applications in adhesives, pharmaceuticals, agriculture, fine and specialty chemicals, absorbents, coating materials, and paints. Acid anhydrides, in particular (meth)acrylic anhydrides, may also be used in polymerization reactions or as crosslinking agents.

Due to their high instability and sensitivity towards hydrolysis, side reactions or polymerization, the manufacturing and use of acid anhydrides, and in particular (meth)acrylic anhydrides, on industrial scale is not always satisfactory.

Partial solutions are described in US Pat. No. 4,857,239 (Hurtel et al.), in US 2002/0161260 A1 (Schmitt et al.), US 2003/0018217 A1 (Dupont et al.), US 2010/0317892 A1 (Paul et al.), US 2011/0137072 A1 (Ansai et al.), which disclose the manufacturing of (meth)acrylic anhydrides by using a transanhydrification reaction between (meth)acrylic acid and a suitable acid anhydride. Another partial solution is described in US Pat. No. 5,491,244 (Ayorinde et al.), which discloses the manufacturing of (meth)acrylic anhydrides via reaction between an aromatic acid chloride and carboxylate ions of acrylic acid and methacrylic acid.

The disclosed methods are not fully satisfactory for the manufacturing of acid anhydrides, in particular (meth)acrylic anhydrides, as they typically involve the forming of unwanted by-products or polymerization products. These undesired side-products not only require additional processing steps, such as extraction steps by distillation, but may also require using additional reagents such as e.g. polymerization inhibitors, catalysts or stabilizers, which then substantially increases the complexity and overall cost of the manufacturing processes.

Without contesting the technical advantages associated with the manufacturing processes known in the art, there is still a need for a process for the manufacturing of (meth)acrylic anhydrides, which overcomes the above-described deficiencies.

Other advantages of the process of the disclosure will be apparent from the following description.

### Summary

According to one aspect, the present disclosure relates to a process for the manufacturing of a (meth)acrylic anhydride, wherein the process comprises the steps of:
A. providing a flow reactor comprising a reaction chamber;
B. providing reactants and reagents comprising:
   a) a (meth)acryloyl halide;
   b) an organic solvent;
   c) (meth)acrylic acid;
   d) and either:
      i. a tertiary amine; or
      ii. an inorganic base and a polar solvent; and
C. incorporating the reactants and reagents into the reaction chamber of the flow reactor, thereby forming a reaction product stream comprising the (meth)acrylic anhydride.

In another aspect, the present disclosure is directed to the use of a polar solvent for the manufacturing of a (meth)acrylic anhydride in a flow reactor.

### Detailed description

According to one aspect, the present disclosure relates to a process for the manufacturing of a (meth)acrylic anhydride, wherein the process comprises the steps of:
A. providing a flow reactor comprising a reaction chamber;
B. providing reactants and reagents comprising:
   a) a (meth)acryloyl halide;
   b) an organic solvent;
   c) (meth)acrylic acid;
   d) and either:
      i. a tertiary amine; or
      ii. an inorganic base and a polar solvent; and
C. incorporating the reactants and reagents into the reaction chamber of the flow reactor, thereby forming a reaction product stream comprising the (meth)acrylic anhydride.

In the context of the present disclosure, it has been surprisingly found that a process as described above provides an efficient, safe, simple, versatile and highly selective method for the manufacturing of (meth)acrylic anhydrides.

Advantageously, the process as described above is a robust and production-efficient process. The process of the present disclosure further provides excellent control of the reaction temperature profile (efficient thermal management), in particular through ensuring rapid and homogeneous mixing, as well as efficient transport of the starting material and intermediate reaction mixtures during the reaction process. As such, the process of the present disclosure allows using a broad scope of possible starting reactants and reagents for the manufacturing of (meth)acrylic anhydrides.

In some other advantageous aspects, the process as described above is able to provide high yields of (meth)acrylic anhydrides having excellent purity and quality due to the suppression or substantial reduction of side reactions such as e.g. mixed anhydrides, degradation products of the (meth)acrylic anhydrides, oligo- or polymerization of the (meth)acrylic anhydrides or Michael addition of acrylic on the acrylic anhydrides.

In some beneficial aspects, the process of the present disclosure does not lead to the forming of hazardous gaseous by-products such as for example hydrogen chloride, carbon dioxide, carbon monoxide or sulphur dioxide, which are known to interfere not only in the flow fluidics and the mixing of the reactants, but also in the reaction chemistry of the processes known in the art.

In some advantageous aspects of the present disclosure, the process for the manufacturing of a (meth)acrylic anhydride may be conducted in presence of a polar solvent such as water in order to provide a two-phase reaction system. Such a two-phase system allows for easy separation of the reaction products, that are present in the organic phase, from those present in the water phase. In the context of the present disclosure, it has surprisingly been found that the use of a polar solvent such as water in the reaction chamber does not have a detrimental influence on the reaction yield, despite the high water-sensitivity of reactants such as (meth)acryloyl halide or the (meth)acrylic anhydride formed during the process of the present disclosure.

Without wishing to be bound by theory, it is believed that these excellent properties are due in particular to the specific combination of the use of a flow reactor and the use of specific reactants and reagents as mentioned above.

In the context of the present disclosure, the term "(meth)acryloyl halide" is meant to refer to acryloyl chloride and methacryloyl chloride (also referred to as 2-methylacryloyl chloride), acryloyl bromide and methacryloyl bromide.

The term "addition stream" is meant to refer to reactants (such as e.g. the (meth)acrylic acid and the (meth)acryloyl halide), the solvents and reagents (such as e.g. the tertiary amine or the inorganic base) flowing from an entry location to the reaction chamber of the flow reactor.

The term "reaction chamber" is meant to refer to a region or area of the flow reactor where separate incoming addition streams are combined and contact one another. The reactants of the addition streams mix and chemically react with one another thereby forming a reaction product stream.

In the context of the present disclosure, the term "flow speed" is meant to refer to the speed (in ml/min) at which the addition streams are incorporated into the reaction chamber of the flow reactor.

The term "residence time" is meant to refer to the period of time the reaction product stream remains in the reaction chamber of the flow reactor from the moment the reactants, and in particular the first addition stream, the second addition stream and the optional third addition stream are incorporated and mixed into the reaction chamber of the flow reactor until the moment the reaction product stream exits the reaction chamber.

In the context of the present disclosure, the expression "molar ratio of compound X to compound Y" is meant to refer to the ratio of moles used of compound X relative to the moles used of compound Y. The calculation of the molar ratio of two compounds is well within the capabilities of those skilled in the art.

In the context of the present disclosure still, the expression "conversion rate of the (meth)acrylic acid into the (meth)acrylic anhydride" is meant to refer to the molar percentage of the (meth)acrylic acid actually converted into the corresponding (meth)acrylic anhydride, as determined by ¹H NMR spectroscopy on the unpurified reaction mixture.

The process of the present disclosure comprises, as a first technical feature, the step of providing a flow reactor comprising a reaction chamber.

Flow reactors for use herein are not particularly limited. Any flow reactor comprising a reaction chamber commonly known in the art may be used in the context of the present disclosure. Suitable flow reactors for use herein will be easily identified by those skilled in the art, in the light of the present description.

Exemplary flow reactors comprising a reaction chamber for use herein are described for example in US 2010/0185013 A1 (Pinnow et al.), WO 2017147040 (Dams et al.), US 2011/0071307 A1 (Ishiyama et al.) and US 2011/0087041 A1 (Ishiyama et al.). Moreover, flow reactors and technologies have been documented in Chem. Commun., 2011, 47, 6512-6535 (Charlotte Wiles and Paul Watts).

Suitable flow reactors for use herein are commercially available, for example, under the trade designation IDEX 91 (ACHROM, Belgium) and LABTRIX START 1805-L-2 (Chemtrix BV, UK), the latter of which can be fitted with a glass microchip, such as those available under the trade designation TYPE 3223 (Chemtrix BV), which can function as the reaction chamber.

Alternative flow reactors for use herein may be built of PFA-tubing with an inner diameter of for example 0.50 mm and a total volume of for example 0.5 ml. Suitable PFA-tubing for use herein are available under the trade designation "IDEX 1512L" from Achrom, Belgium. These alternative flow reactors may be suitably connected to syringe pumps commercially available, for example, under the trade designation Fusion Touch or Fusion Classic from Chemtrix BV, delivering at least two reactant streams from at least two gas-tight syringes, available under the trade designation "Hamilton Syringe 10 ml 1000 series GASTIGHT" available from Hamilton, through PFA tubing with an inner diameter of 1.0 mm, available under the trade designation "IDEX 1507" from Achrom, Belgium, to the reaction chamber of the flow reactor with fittings and luer lock connections, available under the trade designations "IDEX P-628 and IDEX XP-245X" from Achrom, Belgium.

In a typical aspect, the flow reactors for use herein will have various addition ports for adding reactants through additions streams to the reaction chamber of the flow reactor. In many cases, only two, three, four, or five addition ports are used for adding material to the reaction chamber. When there are unused addition ports, the unused addition ports will typically be plugged so as to prevent the intake of any unwanted substances from outside the reaction chamber. One or more of the addition ports can have a check valve to prevent backflow, but this is in most cases not needed because the pressure of the reactant stream through the addition port is usually sufficient to prevent backflow, and because essentially no gaseous products are formed in the process of the present disclosure. The reaction chamber of the flow reactor will also typically have at least one exit port for a product stream to exit.

In a particular aspect, the flow reactor can be a microreactor, wherein the reaction chamber of the flow reactor for use herein has typically an internal volume of no greater than 5 ml, no greater than 1 ml, no greater than 800 microlitres, no greater than 600 microlitres, no greater than 500 microlitres, no greater than 400 microlitres, no greater than 300 microlitres, no greater than 250 microlitres, no greater than 200 microlitres, no greater than 150 microlitres, no greater than 100 microlitres, or even no greater than 50 microlitres.

In another particular aspect, the reaction chamber of the flow reactor has an internal volume of no greater than 500 ml, no greater than 400 ml, no greater than 300 ml, no greater than 200 ml, no greater than 150 ml, no greater than 100 ml, no greater than 80 ml, no greater than 60 ml, no greater than 40 ml, no greater than 20 ml, or even no greater than 10 ml.

The flow reactors for use herein typically have a reaction chamber that has a geometry for promoting mixing of the reactants and reagents added to the reaction chamber. In many cases, the mixing chamber can be designed to create a flowing plug of reactants and reagents such that back-mixing of materials in the flow reactor with materials later added to the flow reactor is mitigated. The reaction chamber can have any suitable geometry, such as a T-shape, star-shape, or circuitous tube shape.

In one particular aspect of the process of the present disclosure, the reactants and reagents comprise:
a) a (meth)acryloyl halide;
b) an organic solvent;
c) (meth)acrylic acid; and
d) a tertiary amine.

According to one particular aspect, the process of the present disclosure further comprises the steps of:
A. providing a first addition stream comprising the (meth)acrylic acid, the tertiary amine and the organic solvent;
B. providing a second addition stream comprising the (meth)acryloyl halide; and
C. incorporating the first addition stream and the second addition stream into the reaction chamber of the flow reactor, thereby forming a reaction product stream comprising the (meth)acrylic anhydride.

According to another particular aspect, the process of the present disclosure comprises the steps of:
A. providing a first addition stream comprising the (meth)acrylic acid;
B. providing a second addition stream comprising the tertiary amine and the organic solvent;
C. incorporating the first addition stream and the second addition stream into the reaction chamber of a first flow reactor, thereby forming an intermediate reaction product stream comprising a (meth)acrylic acid salt, in particular a (meth)acrylic acid-tertiary amine salt;
D. providing a third addition stream comprising the intermediate reaction product stream comprising the (meth)acrylic acid salt;
E. providing a fourth addition stream comprising the (meth)acryloyl halide; and
F. incorporating the third addition stream and the fourth addition stream into the reaction chamber of a second flow reactor, thereby forming a reaction product stream comprising the (meth)acrylic anhydride.

According to still another particular aspect, the process of the present disclosure comprises the steps of:
A. providing a first addition stream comprising the (meth)acrylic acid;
B. providing a second addition stream comprising the tertiary amine;
C. providing a third addition stream comprising the organic solvent;
D. incorporating the first addition stream, the second addition stream and the third addition stream into the reaction chamber of a first flow reactor, thereby forming an intermediate reaction product stream comprising a (meth)acrylic acid salt, in particular a (meth)acrylic acid-tertiary amine salt;
E. providing a fourth addition stream comprising the intermediate reaction product stream comprising the (meth)acrylic acid salt;
F. providing a fifth addition stream comprising the (meth)acryloyl halide; and
G. incorporating the fourth addition stream and the fifth addition stream into the reaction chamber of a second flow reactor, thereby forming a reaction product stream comprising the (meth)acrylic anhydride.

According to yet another particular aspect of the process of the present disclosure, the (meth)acryloyl halide is provided by reacting the (meth)acrylic acid with a halogenating agent and a co-agent in the reaction chamber of a secondary flow reactor thereby forming the (meth)acryloyl halide for use in the process as described above.

According to this particular aspect of the disclosure, the process further comprises the steps of:
A. providing a first addition stream comprising the (meth)acrylic acid, the co-agent and optionally, a solvent;
B. providing a second addition stream comprising the halogenating agent;
C. incorporating the first addition stream and the second addition stream into the reaction chamber of a first flow reactor, thereby forming an intermediate reaction product stream comprising the (meth)acryloyl halide and (meth)acrylic acid;
D. providing a third addition stream comprising the intermediate reaction product stream comprising the (meth)acryloyl halide and (meth)acrylic acid;
E. providing a fourth addition stream comprising, the tertiary amine and the organic solvent; and
F. incorporating the third addition stream and the fourth addition stream into the reaction chamber of a second flow reactor, thereby forming a reaction product stream comprising the (meth)acrylic anhydride.

In an alternative aspect of the process of the present disclosure, the reactants and reagents comprise:
a) a (meth)acryloyl halide;
b) an organic solvent;
c) (meth)acrylic acid;
d) an inorganic base;
e) a polar solvent; and
f) optionally, a phase transfer catalyst.

According to this particular aspect of the disclosure, the process further comprises the steps of:
A. providing a first addition stream comprising the (meth)acrylic acid, the inorganic base, the polar solvent and optionally, the phase transfer catalyst;
B. providing a second addition stream comprising the (meth)acryloyl halide and the organic solvent; and
C. incorporating the first addition stream and the second addition stream into the reaction chamber of the flow reactor, thereby forming a reaction product stream comprising the (meth)acrylic anhydride.

In another particular aspect of the disclosure, the process further comprises the steps of:
A. providing a first addition stream comprising the (meth)acrylic acid;
B. providing a second stream comprising the inorganic base and the polar solvent;
C. providing a third stream comprising the optional phase transfer catalyst and the polar solvent;
D. providing a fourth addition stream comprising the (meth)acryloyl halide;
E. providing a fifth addition stream comprising the organic solvent; and
F. incorporating the first addition stream, the second addition stream, the third addition stream, the fourth addition stream, and the fifth addition stream into the reaction chamber of the flow reactor, thereby forming a reaction product stream comprising the (meth)acrylic anhydride.

As will be easily apparent to those skilled in the art, the flow reactor(s) for use herein may comprise various addition ports for the incorporation of various reagent/reactant addition streams into the reaction chamber(s). The various reactant addition streams may be incorporated into the reaction chamber(s) through distinct or common addition ports. Also, the various reactant addition streams may be incorporated into the reaction chamber(s) simultaneously or at distinct addition times.

In an exemplary aspect, the flow reactor(s) further comprise at least as many addition ports as addition streams.

In one particular aspect of the process, some or all of the additions streams comprising the reactants and reagents are pre-mixed prior to incorporation into the reaction chamber(s) of the flow reactor(s).

In another particular aspect of the process, some or all of the additions streams comprising the reactants and reagents are incorporated simultaneously into the reaction chamber(s) of the flow reactor(s).

According to a typical aspect of the process of the present disclosure, some or all of the additions streams comprising the reactants and reagents are incorporated into the reaction chamber(s) of the flow reactor(s) in successive steps.

In practice, the various reactant addition streams are incorporated and allowed to combine and contact one another to chemically react with one another in the reaction chamber(s) of the flow reactor(s), thereby forming a reaction product stream comprising the (meth)acrylic anhydride.

In one exemplary aspect of the process according to the disclosure, the addition streams comprising the reactants and reagents are incorporated and combined into the reaction chamber(s) of the flow reactor(s), thereby forming a reaction product stream comprising the (meth)acrylic anhydride.

The temperature of the addition streams and the temperature of the reaction chamber(s) for use herein will be easily identified by those skilled in the art, in the light of the present description.

In an advantageous aspect of the process, the temperature of the addition streams comprising the reactants and reagents is such that the addition streams are liquid prior to incorporation into the reaction chamber(s) of the flow reactor(s). In an alternative aspect, the temperature of the addition streams comprising the reactants and reagents is such that the addition streams are at least flowable/pumpable through conventional addition pumps prior to incorporation into the reaction chamber(s) of the flow reactor(s).

According to a typical aspect of the process of the present disclosure, the temperature of at least one of the first addition stream, the second addition stream, the third addition stream, the fourth addition stream, and the optional fifth addition stream is in range from 0°C to 120°C, from 0°C to 100°C, from 0°C to 80°C, from 5°C to 60°C, from 10°C to 55°C, from 15°C to 45°C, from 20°C to 35°C, or even from 20°C to 25°C, prior to incorporation into the reaction chamber(s) of the flow reactor(s).

According to another typical aspect of the process, the temperature of the reaction chamber(s) of the flow reactor(s) is in a range from 10°C to 100°C, from 10°C to 80°C, from 10°C to 70°C, from 10°C to 60°C, from 15°C to 60°C, from 15°C to 55°C, from 15°C to 50°C, from 15°C to 40°C, or even from 20°C to 30°C, after incorporation of the reactants and reagents, and in particular the first addition stream, the second addition stream, the third addition stream, the fourth addition stream, and the optional fifth addition stream into the reaction chamber of the flow reactor(s).

In still a further typical aspect, the temperature of the reaction chamber(s) of the flow reactor(s) is no greater than 100°C, no greater than 80°C, no greater than 60°C, no greater than 50°C, or even no greater than 40°C, after incorporation of the reactants and reagents, and in particular the first addition stream, the second addition stream, the third addition stream, the fourth addition stream, and the optional fifth addition stream into the reaction chamber(s) of the flow reactor(s).

In a further advantageous aspect of the process, the addition streams may be added at room temperature (23°C +/- 2°C) and the reaction chamber(s) is not cooled during the manufacturing process.

According to another beneficial aspect of the process, the flow reactor(s) for use herein is not temperature controlled during the process, in particular not cooled by cooling equipment.

According to still another beneficial aspect of the process, the reaction chamber(s) of the flow reactor(s) is not temperature controlled during the process, in particular not cooled by cooling equipment.

The various addition streams may be incorporated into the reaction chamber(s) of the flow reactor(s) using any means commonly known in the art. In a particular aspect, the first addition stream, the second addition stream, the third addition stream, the fourth addition stream, and the optional fifth addition stream are incorporated into the reaction chamber(s) by using suitable (high) pressure pumps, such as rotary pumps, screw pumps, plunger plumps, gear pumps, peristaltic pumps, syringe pumps or piston pumps.

The flow speed of the various addition streams for use herein is not particularly limited. Suitable flow speeds for use herein will be easily identified by those skilled in the art, in the light of the present description. In particular, the flow speed of the various addition streams for use herein may be appropriately chosen such that the molar ratios between the different reactants and reagents is according to the process and maintained constant throughout the process.

In an advantageous aspect of the process, the first addition stream, the second addition stream, the third addition stream, the fourth addition stream, and the fifth addition stream are incorporated into the reaction chamber(s) of the flow reactor(s) each at a flow speed in a range from 0.01 ml/min to 100 ml/min, 0.01 ml/min to 80 ml/min, 0.05 ml/min to 60 ml/min, 0.08 ml/min to 50 ml/min, from 0.1 ml/ min to 40 ml/min, from 0.1 ml/min to 20 ml/min, or even from 0.1 ml/min to 10 ml/min.

The residence time of the reaction product stream comprising the (meth)acrylic anhydride in the reaction chamber of the flow reactor is typically chosen in function of the time required to obtain the desired yield and purity of the resulting (meth)acrylic anhydride. The residence time is advantageously chosen to avoid any potential further reaction of the (meth)acrylic anhydride, including for example, hydrolysis or homopolymerization reactions.

Suitable residence times for use herein will be easily identified by those skilled in the art, in the light of the present description.

According to a beneficial aspect of the process of the present disclosure, the residence time of the reaction product stream comprising the (meth)acrylic anhydride in the reaction chamber(s) of the flow reactor(s) is in a range from 1 to 1800 seconds, from 1 to 1500 seconds, from 1 to 1200 seconds, from 5 to 1000 seconds, from 10 to 900 seconds, from 15 to 720 seconds, from 20 to 600 seconds, from 30 to 480 seconds, from 30 to 360 seconds, from 60 to 360 seconds, from 60 to 300 seconds, or even from 60 to 240 seconds.

In another beneficial aspect of the process, the residence time of the reaction product stream comprising the (meth)acrylic anhydride in the reaction chamber of the flow reactor(s) is no greater than 1800 seconds, no greater than 1500 seconds, no greater than 1200 seconds, no greater than 1000 seconds, no greater than 900 seconds, no greater than 720 seconds, no greater than 600 seconds, no greater than 480 seconds, no greater than 360 seconds, no greater than 300 seconds, or even no greater than 240 seconds.

The reactants for use in the process according to the present disclosure, comprise a (meth)acryloyl halide. Suitable (meth)acryloyl halides for use herein will be easily identified by those skilled in the art, in the light of the present description.

According to a typical aspect of the process, the (meth)acryloyl halide for use herein is a (meth)acryloyl chloride or a (meth)acryloyl bromide, preferably a (meth)acryloyl chloride.

In one advantageous aspect, (meth)acryloyl halide for use herein is acryloyl chloride or methacryloyl chloride, preferably acryloyl chloride.

Acryloyl chloride and methacryloyl chloride are readily and commercially available for example from ABCR, Germany or may alternatively be prepared in-situ as described in some aspects of the disclosure.

The reagents for use in the process according to the present disclosure, further comprise an organic solvent. Organic solvents for use herein are not particularly limited. Suitable organic solvents for use herein will be easily identified by those skilled in the art, in the light of the present description.

Suitable organic solvents for use herein may advantageously have limited or substantially no solubility or miscibility with the polar solvent for use in some other aspects of the disclosure. In those aspects where both an organic and a polar solvent are part of the reactants and reagents for use in the process according to the disclosure, a two-phase system is advantageously formed.

In a beneficial aspect, the organic solvent for use herein is selected from the group consisting of aliphatic or aromatic hydrocarbons, ethers, amides, sulfoxides and halogenated solvents, and any mixtures thereof.

In a preferred aspect, the organic solvent for use herein is selected from the group consisting of halogenated hydrocarbons, in particular chlorinated or brominated organic solvents, preferably chlorinated organic solvents.

In a particularly preferred aspect, the organic solvent for use herein is selected to comprise dichloromethane.

In some executions of the process of the present disclosure, the reagents for use herein may further comprise a tertiary amine. Tertiary amines for use herein are not particularly limited. Suitable tertiary amines for use herein will be easily identified by those skilled in the art, in the light of the present description.

Suitable tertiary amines for use herein may advantageously be selected such that the corresponding tertiary amine halide salt formed during the process according to some aspects of the disclosure remain substantially soluble in the reaction product stream formed by incorporating the reactants and reagents into the reaction chamber of the flow reactor.

According to an advantageous aspect, the tertiary amine for use herein comprises at least one of triisopropylamine, diisopropylethylamine, triethyl amine, trimethyl amine, methyldiethyl amine, and any mixtures thereof.

According to a preferred aspect, the tertiary amine for use herein comprises diisopropylethylamine or triethyl amine, preferably diisopropylethylamine.

In some particular executions of the process of the present disclosure, the reactants for use herein may further comprise a halogenating agent. Halogenating agents for use herein are not particularly limited. Any halogenating agent commonly known in the art may be used in the context of the present disclosure. Suitable halogenating agents for use herein will be easily identified by those skilled in the art, in the light of the present description.

According to an advantageous aspect of the process, the halogenating agent for use herein is selected from the group of chlorinating agents or brominating agents, preferably chlorinating agents.

According to another advantageous aspect of the process, the halogenating agent is a chlorinating agent preferably selected from the group consisting of thionyl chloride, phosphoryl chloride, oxalyl chloride, and any mixtures thereof.

According to still another advantageous aspect of the process, the halogenating agent for use herein is a brominating agent preferably selected from the group consisting of thionyl bromide, phosphoryl bromide, and any mixtures thereof.

In one preferred aspect of the process, the halogenating agent is selected from the group consisting of thionyl chloride, phosphoryl chloride, oxalyl chloride, thionyl bromide, phosphoryl bromide, and any mixtures thereof.

In another preferred aspect of the process, the halogenating agent for use herein is selected from the group consisting of thionyl chloride, phosphoryl chloride, oxalyl chloride, and any mixtures thereof.

In a more preferred aspect of the process, the halogenating agent is selected from the group consisting of thionyl chloride, phosphoryl chloride, and any mixtures thereof.

In a particularly preferred aspect of the process, the halogenating agent is selected to be phosphoryl chloride.

Suitable halogenating agent for use herein may advantageously be selected such that they do not lead to the formation of hazardous gaseous by-products such as hydrogen chloride, carbon monoxide or sulphur dioxide.

In some particular executions of the process of the present disclosure, the reactants for use herein may comprise a reaction co-agent to facilitate the halogenation reaction of the (meth)acrylic acid with the halogenating agent. According to this specific execution of the process of the present disclosure, the (meth)acryloyl halide for use herein is conveniently produced *in-situ* in the reaction chamber of the flow reactor for further reaction with the remaining of the (meth)acrylic acid.

According to the advantageous aspect of the process according to which the (meth)acryloyl halide for use herein is conveniently produced *in-situ* in the reaction chamber of the flow reactor, the co-agent for use herein is selected from the group consisting of N,N-disubstituted amides. More advantageously, the co-agent for use in some aspect of the process is selected from the group consisting of linear N,N-disubstituted amides, cyclic N,N-disubstituted amides, heterocyclic N,N-disubstituted amides, and any combinations or mixtures thereof.

According to another advantageous aspect, the co-agent is selected from the group consisting of N,N-disubstituted heterocyclic amides and N,N-dialkyl amides, wherein the alkyl group is preferably selected from the group of methyl, ethyl, propyl and butyl.

In a beneficial aspect, the co-agent for use herein is selected from the group consisting of N,N-dialkyl formamides and N,N-dialkyl acetamides, wherein the alkyl group is preferably selected from the group of methyl, ethyl, propyl and butyl.

In another beneficial aspect, the co-agent for use herein is selected from the group consisting of N,N-disubstituted heterocyclic amides, for example N-formyl morpholine.

In still another beneficial aspect, the co-agent is selected from the group consisting of N,N-dimethyl formamide, N,N-diethyl formamide, N,N-dimethyl acetamide, N-formyl morpholine, and any combinations or mixtures thereof.

According to a particularly beneficial aspect, the co-agent is selected from the group consisting of N,N-dimethyl formamide, N,N-diethyl formamide, N-formyl morpholine, and any combinations or mixtures thereof.

According to a particularly preferred aspect, the co-agent for use herein is selected to be N,N-dimethyl formamide.

According to a typical aspect of the process, the co-agent for use herein is different from the tertiary amine as described above.

In some executions of the process of the present disclosure, the reagents for use herein may further comprise an inorganic base. Suitable inorganic bases for use herein will be therefore easily identified by those skilled in the art, in the light of the present description.

Suitable inorganic bases for use herein may advantageously be selected such that they remain substantially soluble in the polar solvent for use in the process according to some aspects of the disclosure.

According to an advantageous aspect of the process, the inorganic base for use herein is selected from the group consisting of alkali- or alkali earth metal hydroxides, in particular alkali metal hydroxides.

According to another advantageous aspect of the process, the inorganic base for use herein is selected from the group consisting of sodium hydroxide, potassium hydroxide and lithium hydroxide.

According to a particularly preferred aspect, the inorganic base for use herein is sodium hydroxide or potassium hydroxide, preferably sodium hydroxide.

In some executions of the process of the present disclosure, the reagents for use herein may further comprise a polar solvent. Polar solvents for use herein are not particularly limited. Polar solvents for use herein will be easily identified by those skilled in the art, in the light of the present description.

Suitable polar solvents for use herein may advantageously be selected such that they are able to substantially dissolve the inorganic base and the phase transfer catalyst used in some aspects of the disclosure, as well as the inorganic salts formed during the process according to some aspects of the disclosure.

Suitable polar solvents for use herein may also beneficially have limited or substantially no solubility or miscibility with the organic solvent.

According to an advantageous aspect of the process, the polar solvent for use herein is selected from the group consisting of water, alcohols, amides, sulfoxides, and any mixtures thereof; and wherein the polar solvent is different from the organic solvent.

According to a particularly preferred aspect, the polar solvent is or comprises water.

In some particular executions of the process of the present disclosure, the reactants and reagents for use herein may further comprise a phase transfer catalyst. Phase transfer catalysts for use herein are not particularly limited. Any phase transfer catalysts commonly known in the art to facilitate the transport of active reactants and reagents between a polar solvent and an organic solvent, may be used in the context of the present disclosure. Suitable phase transfer catalysts for use herein will be therefore easily identified by those skilled in the art, in the light of the present description.

According to an advantageous aspect of the process, the phase transfer catalyst for use herein is selected from the group consisting of salts of tertiary amines, in particular hydrogen halide salts of triethyl amine, trimethyl amine; and quaternary ammoniums salts, in particular tetraethyl ammonium halides, tetramethylammonium halides, tetraisopropylammonium halides, tetrabutylammonium halides; and any mixtures thereof.

According to another advantageous aspect of the process, the phase transfer catalyst for use herein is selected from the group consisting of tetraethyl ammonium halides and tetrabutylammonium halides.

According to a particularly advantageous aspect, the phase transfer catalyst is selected from the group consisting of hydrogen halide salts of triethyl amine and tetrabutylammonium halides, in particular hydrogen chloride salt of triethyl amine and tetrabutylammonium bromide.

In a typical aspect of the process according to the disclosure, the amount of the phase transfer catalyst is in a range of from 5 to 50 mol %, from 5 to 45 mol %, from 7 to 45 mol %, from 7 to 40 mol %, from 7 to 35 mol %, from 7 to 30 mol %, or even from 10 to 30 mol %, based on the molar equivalent of the (meth)acrylic acid.

According to one advantageous aspect of the process of the present disclosure, the molar ratio of the (meth)acrylic acid to the (meth)acryloyl halide is 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; or even 1 to at least 1.5.

According to another advantageous aspect of the process of the present disclosure, the molar ratio of the (meth)acrylic acid to the (meth)acryloyl halide is no greater than 1 to 1.5; no greater than 1 to 1.4; no greater than 1 to 1.3; or even no greater than 1 to 1.2.

According to still another advantageous aspect of the process of the present disclosure, the molar ratio of the (meth)acrylic acid to the (meth)acryloyl halide is in a range between 1 to 0.8 and 1 to 1.5, between 1 to 1 and 1 to 1.5, between 1 to 1 and 1 to 1.4, between 1 to 1 and 1 to 1.3, or even between 1 to 1 and 1 to 1.2.

According to a preferred aspect of the process of the present disclosure, the molar ratio of the (meth)acrylic acid to the (meth)acryloyl halide is about 1 to 1 or 1 to 1.1.

In another advantageous aspect of the process, the molar ratio of the (meth)acrylic acid to the tertiary amine is 1 to at least 0.8; 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; 1 to at least 1.5; 1 to at least 2; 1 to at least 2.5; or even 1 to at least 3.

In still another advantageous aspect of the process, the molar ratio of the (meth)acrylic acid to the tertiary amine is no greater than 1 to 3; no greater than 1 to 2.5; no greater than 1 to 2; no greater than 1 to 1.5; no greater than 1 to 1.4; no greater than 1 to 1.3; or even no greater than 1 to 1.2.

In a beneficial aspect of the process, the molar ratio of the (meth)acrylic acid to the tertiary amine is in a range between 1 to 0.8 and 1 to 3, between 1 to 0.8 and 1 to 2.5, between 1 to 0.8 and 1 to 2, between 1 to 0.8 and 1 to 1.5, between 1 to 1 and 1 to 1.5, between 1 to 1 and 1 to 1.4, between 1 to 1 and 1 to 1.3, or even between 1 to 1 and 1 to 1.2.

In one preferred aspect of the process of the present disclosure, the molar ratio of the (meth)acrylic acid to the tertiary amine is about 1 to 1 or 1 to 1.5.

In another beneficial aspect of the process, the molar ratio of the halogenating agent to the (meth)acrylic acid is 1 to at least 1.5; 1 to at least 1.6; 1 to at least 1.7; 1 to at least 1.8; 1 to at least 1.9; 1 to at least 2.0; 1 to at least 2.1; or even 1 to at least 2.2.

In still another beneficial aspect of the process, the molar ratio of the halogenating agent to the (meth)acrylic acid is no greater than 1 to 2.5; no greater than 1 to 2.4; no greater than 1 to 2.2; or even no greater than 1 to 2.0.

In still another beneficial aspect of the process, the molar ratio of the halogenating agent to the (meth)acrylic acid is in a range between 1 to 1.5 and 1 to 2.5, between 1 to 1.6 and 1 to 2.4, between 1 to 1.7 and 1 to 2.2, between 1 to 1.8 and 1 to 2.2, between 1 to 1.9 and 1 to 2.2, or even between 1 to 1.9 and 1 to 2.1.

According to another preferred aspect of the process of the present disclosure, the molar ratio of the halogenating agent to the (meth)acrylic acid is about 1 to 2.

In another advantageous aspect of the process, the molar ratio of the halogenating agent to the co-agent is 1 to at least 1; 1 to at least 1.2; 1 to at least 1.4; or even 1 to at least 1.5.

In still another advantageous aspect of the process, the molar ratio of the halogenating agent to the co-agent is no greater than 1 to 2.5; no greater than 1 to 2.4; no greater than 1 to 2.2; no greater than 1 to 2; no greater than 1 to 1.8; no greater than 1 to 1.6; even no greater than 1 to 1.5.

In a beneficial aspect of the process, the molar ratio of the halogenating agent to the co-agent is in a range between 1 to 1 and 1 to 2.5, between 1 to 1 and 1 to 2.2, between 1 to 1.2 and 1 to 2, between 1 to 1.3 and 1 to 1.8, between 1 to 1.3 and 1 to 1.8, or even between 1 to 1.4 and 1 to 1.6.

In another preferred aspect of the process of the present disclosure, the molar ratio of the halogenating agent to the co-agent is about 1 to 1.5.

According to yet another advantageous aspect of the process of the present disclosure, the molar ratio of the (meth)acrylic acid to the inorganic base is in a range between 1 to 0.8 and 1 to 1.1, or even between 1 to 1 and 1 to 1.05.

In an advantageous aspect, the process of the present disclosure does not comprise a transanhydrification step, or is (substantially) free of any transanhydrification step.

As will be apparent to those skilled in the art, the reactants, the reagents and the reaction product streams for use in the present process may comprise optional ingredients commonly known in the art for similar chemical reactions.

According to one advantageous aspect of the process of the disclosure, the addition streams comprise polymerization inhibitors, in particular polymerization inhibitors selected from the group of phenothiazines and hydroquinones, in particular hydroquinone monomethyl ethers and hydroquinone methyl esters.

According to an advantageous aspect of the process of the present disclosure, the reaction product stream comprises the (meth)acrylic anhydride in an amount of at least 80 wt %, at least 85 wt %, at least 90 wt %, at least 95 wt %, or even at least 98 wt % based on the total weight of the (meth)acrylic anhydride, the (meth)acrylic acid, and the organic by-products in the reaction product stream.

The weights of the various components of the product stream can be measured by any suitable means, for example, by gas chromatography or NMR spectroscopy. When gas chromatography is used, the compounds in the product stream can be identified by comparing their residence time to that of standards on the same column. The areas for the peaks can be calculated using standard software, or even manually, and then converted into concentration by using calibration curves. The calibration curves can be established by standard samples having known concentrations of the compounds. Other suitable means of determining the wt % of the various components of the product stream include, liquid chromatography, such as HPLC, and mass spectrometry.

According to another advantageous aspect of the process of the present disclosure, the conversion rate of the (meth)acrylic acid into the (meth)acrylic anhydride is of at least 80 mol %, at least 85 mol %, at least 90 mol %, at least 95 mol %, or even at least 98 mol % based on the molar equivalent of the (meth)acrylic acid or (meth)acrylic acid salt, and when determined by ¹H NMR spectroscopy.

In one advantageous aspect of the process according to the disclosure, the flow reactor is not temperature controlled during the process, in particular not cooled by cooling equipment. In some other advantageous aspects of the process, the reaction chamber of the flow reactor is not temperature controlled either during the process, in particular not cooled by cooling equipment.

This is a particularly surprising characteristic as the reaction between a (meth)acryloyl halide and (meth)acrylic acid, as well as the optional halogenation reaction, more specifically the halogenation reaction of (meth)acrylic acid into (meth)acryloyl halides, are known to be highly exothermic, thus typically requiring external cooling to avoid potentially dangerous release of heat, unwanted side reactions, or both. Surprisingly, the process disclosed herein proceeds in high yields even when performed at room temperature and without necessarily using a cooling device for the reaction chamber of the flow reactor.

According to one beneficial aspect of the present disclosure, the process as described herein may be performed as a continuous process.

According to a particularly advantageous aspect of the process of the disclosure, substantially no gaseous by-products are formed in the reaction chamber of the flow reactor, in particular no gaseous by-products selected from the group of hydrochloric acid, carbon dioxide, carbon monoxide and sulphur dioxide.

In another aspect, the present disclosure relates to the use of a polar solvent for the manufacturing of a (meth)acrylic anhydride in a flow reactor.

In one advantageous aspect of this use, the polar solvent is or comprises water.
Item 1 is a process for the manufacturing of a (meth)acrylic anhydride, wherein the process comprises the steps of:
   A. providing a flow reactor comprising a reaction chamber;
   B. providing reactants and reagents comprising:
      a) a (meth)acryloyl halide;
      b) an organic solvent;
      c) (meth)acrylic acid;
      d) and either:
         i. a tertiary amine; or
         ii. an inorganic base and a polar solvent; and
   C. incorporating the reactants and reagents into the reaction chamber of the flow reactor, thereby forming a reaction product stream comprising the (meth)acrylic anhydride.
Item 2 is a process according to item 1, wherein the reactants and reagents comprise:
   a) a (meth)acryloyl halide;
   b) an organic solvent;
   c) (meth)acrylic acid; and
   d) a tertiary amine.
Item 3 is a process according to item 2, wherein the (meth)acryloyl halide is provided by reacting the (meth)acrylic acid with a halogenating agent and a co-agent in the reaction chamber of a secondary flow reactor thereby forming the (meth)acryloyl halide for use in the process according to any of item 1 or 2.
Item 4 is a process according to item 1, wherein the reactants and reagents comprise:
   a) a (meth)acryloyl halide;
   b) an organic solvent;
   c) (meth)acrylic acid;
   d) an inorganic base;
   e) a polar solvent; and
   f) optionally, a phase transfer catalyst.
Item 5 is a process according to any of item 1 or 2, which comprises the steps of:
   A. providing a first addition stream comprising the (meth)acrylic acid, the tertiary amine and the organic solvent;
   B. providing a second addition stream comprising the (meth)acryloyl halide; and
   C. incorporating the first addition stream and the second addition stream into the reaction chamber of the flow reactor, thereby forming a reaction product stream comprising the (meth)acrylic anhydride.
Item 6 is a process according to any of item 1 or 2, which comprises the steps of:
   A. providing a first addition stream comprising the (meth)acrylic acid;
   B. providing a second addition stream comprising the tertiary amine and the organic solvent;
   C. incorporating the first addition stream and the second addition stream into the reaction chamber of a first flow reactor, thereby forming an intermediate reaction product stream comprising a (meth)acrylic acid salt, in particular a (meth)acrylic acid-tertiary amine salt;
   D. providing a third addition stream comprising the intermediate reaction product stream comprising the (meth)acrylic acid salt;
   E. providing a fourth addition stream comprising the (meth)acryloyl halide; and
   F. incorporating the third addition stream and the fourth addition stream into the reaction chamber of a second flow reactor, thereby forming a reaction product stream comprising the (meth)acrylic anhydride.
Item 7 is a process according to any of item 1 or 2, which comprises the steps of:
   A. providing a first addition stream comprising the (meth)acrylic acid;
   B. providing a second addition stream comprising the tertiary amine;
   C. providing a third addition stream comprising the organic solvent;
   D. incorporating the first addition stream, the second addition stream and the third addition stream into the reaction chamber of a first flow reactor, thereby forming an intermediate reaction product stream comprising a (meth)acrylic acid salt, in particular a (meth)acrylic acid-tertiary amine salt;
   E. providing a fourth addition stream comprising the intermediate reaction product stream comprising the (meth)acrylic acid salt;
   F. providing a fifth addition stream comprising the (meth)acryloyl halide; and
   G. incorporating the fourth addition stream and the fifth addition stream into the reaction chamber of a second flow reactor, thereby forming a reaction product stream comprising the (meth)acrylic anhydride.
Item 8 is a process according to item 3, which comprises the steps of:
   A. providing a first addition stream comprising the (meth)acrylic acid, the co-agent and optionally, a solvent;
   B. providing a second addition stream comprising the halogenating agent;
   C. incorporating the first addition stream and the second addition stream into the reaction chamber of a first flow reactor, thereby forming an intermediate reaction product stream comprising the (meth)acryloyl halide and (meth)acrylic acid;
   D. providing a third addition stream comprising the intermediate reaction product stream comprising the (meth)acryloyl halide and (meth)acrylic acid;
   E. providing a fourth addition stream comprising, the tertiary amine and the organic solvent; and
   F. incorporating the third addition stream and the fourth addition stream into the reaction chamber of a second flow reactor, thereby forming a reaction product stream comprising the (meth)acrylic anhydride.
Item 9 is a process according to item 4, which comprises the steps of:
   A. providing a first addition stream comprising the (meth)acrylic acid, the inorganic base, the polar solvent and optionally, the phase transfer catalyst;
   B. providing a second addition stream comprising the (meth)acryloyl halide and the organic solvent; and
   C. incorporating the first addition stream and the second addition stream into the reaction chamber of the flow reactor, thereby forming a reaction product stream comprising the (meth)acrylic anhydride.
Item 10 is a process according to item 4, which comprises the steps of:
   A. providing a first addition stream comprising the (meth)acrylic acid;
   B. providing a second stream comprising the inorganic base and the polar solvent;
   C. providing a third stream comprising the optional phase transfer catalyst and the polar solvent;
   D. providing a fourth addition stream comprising the (meth)acryloyl halide;
   E. providing a fifth addition stream comprising the organic solvent; and
   F. incorporating the first addition stream, the second addition stream, the third addition stream, the fourth addition stream, and the fifth addition stream into the reaction chamber of the flow reactor, thereby forming a reaction product stream comprising the (meth)acrylic anhydride.
Item 11 is a process according to any of the preceding items, wherein the flow reactor(s) further comprise at least a first addition port, a second addition port, a third addition port, a fourth addition port, and optionally a fifth addition port, and wherein the first addition stream is incorporated into the reaction chamber of the flow reactor through the first addition port, the second addition stream is incorporated through the second addition port, the third addition stream is incorporated through the third addition port, the fourth addition stream is incorporated through the fourth addition port, and the optional fifth addition stream is incorporated through the optional fifth addition port.
Item 12 is a process according to any of the preceding items, wherein some or all of the addition streams comprising the reactants and reagents are pre-mixed prior to incorporation into the reaction chamber of the flow reactor(s).
Item 13 is a process according to any of the preceding items, wherein some or all of the addition streams comprising the reactants and reagents are incorporated simultaneously into the reaction chamber of the flow reactor(s).
Item 14 is a process according to any of the preceding items, wherein some or all of the addition streams comprising the reactants and reagents are incorporated into the reaction chamber of the flow reactor(s) in successive steps.
Item 15 is a process according to any of the preceding items, wherein some or all of the addition streams comprising the reactants and reagents are incorporated and combined into the reaction chamber of the flow reactor, thereby forming a reaction product stream comprising the (meth)acrylic anhydride.
Item 16 is a process according to any of the preceding items, wherein the first addition stream, the second addition stream, the third addition stream, the fourth addition stream, and the fifth addition stream are incorporated into the reaction chamber of the flow reactor each at a flow speed in a range from 0.01 ml/min to 100 ml/min, 0.01 ml/min to 80 ml/min, 0.05 ml/min to 60 ml/min, 0.08 ml/min to 50 ml/min, from 0.1 ml/ min to 40 ml/min, from 0.1 ml/min to 20 ml/min, or even from 0.1 ml/min to 10 ml/min.
Item 17 is a process according to any of the preceding items, wherein the addition streams comprising reactants and reagents are liquid prior to incorporation into the reaction chamber of the flow reactor(s).
Item 18 is a process according to any of the preceding items, wherein the temperature of the addition streams, in particular at least one of the first addition stream, the second addition stream, the third addition stream, the fourth addition stream, and the optional fifth addition stream is in range from 0°C to 120°C, from 0°C to 100°C, from 0°C to 80°C, from 5°C to 60°C, from 10°C to 55°C, from 15°C to 45°C, from 20°C to 35°C, or even from 20°C to 25°C, prior to incorporation into the reaction chamber of the flow reactor(s).
Item 19 is a process according to any of the preceding items, wherein the temperature of the reaction chamber of the flow reactor(s) is in a range from 10°C to 100°C, from 10°C to 80°C, from 10°C to 70°C, from 10°C to 60°C, from 15°C to 60°C, from 15°C to 55°C, from 15°C to 50°C, from 15°C to 40°C, or even from 20°C to 30°C, after incorporation of the addition streams comprising the reactants and reagents, and in particular the first addition stream, the second addition stream, the third addition stream, the fourth addition stream, and the optional fifth addition stream into the reaction chamber of the flow reactor(s).
Item 20 is a process according to any of the preceding items, wherein the temperature of the reaction chamber of the flow reactor(s) is no greater than 100°C, no greater than 80°C, no greater than 60°C, no greater than 50°C, or even no greater than 40°C, after incorporation of the reactants and reagents, and in particular the first addition stream, the second addition stream, the third addition stream, the fourth addition stream, and the optional fifth addition stream into the reaction chamber of the flow reactor(s).
Item 21 is a process according to any of the preceding items, wherein the flow reactor(s) is not temperature controlled during the process, in particular not cooled by cooling equipment.
Item 22 is a process according to any of the preceding items, wherein the reaction chamber of the flow reactor(s) is not temperature controlled during the process, in particular not cooled by cooling equipment.
Item 23 is a process according to any of the preceding items, wherein the residence time of the reaction product stream comprising the (meth)acrylic anhydride in the reaction chamber of the flow reactor(s) is in a range from 1 to 1800 seconds, from 1 to 1500 seconds, from 1 to 1200 seconds, from 5 to 1000 seconds, from 10 to 900 seconds, from 15 to 720 seconds, from 20 to 600 seconds, from 30 to 480 seconds, from 30 to 360 seconds, from 60 to 360 seconds, from 60 to 300 seconds, or even from 60 to 240 seconds.
Item 24 is a process according to any of the preceding items, wherein the residence time of the reaction product stream comprising the (meth)acrylic anhydride in the reaction chamber of the flow reactor(s) is no greater than 1800 seconds, no greater than 1500 seconds, no greater than 1200 seconds, no greater than 1000 seconds, no greater than 900 seconds, no greater than 720 seconds, no greater than 600 seconds, no greater than 480 seconds, no greater than 360 seconds, no greater than 300 seconds, or even no greater than 240 seconds.
Item 25 is a process according to any of the preceding items, wherein the reaction chamber of the flow reactor(s) has an internal volume of no greater than 5 ml, no greater than 1 ml, no greater than 800 microlitres, no greater than 600 microlitres, no greater than 500 microlitres, no greater than 400 microlitres, no greater than 300 microlitres, no greater than 250 microlitres, no greater than 200 microlitres, no greater than 150 microlitres, no greater than 100 microlitres, or even no greater than 50 microlitres.
Item 26 is a process according to any of items 1 to 24, wherein the reaction chamber of the flow reactor has an internal volume of no greater than 500 ml, no greater than 400 ml, no greater than 300 ml, no greater than 200 ml, no greater than 150 ml, no greater than 100 ml, no greater than 80 ml, no greater than 60 ml, no greater than 40 ml, no greater than 20 ml, or even no greater than 10 ml.
Item 27 is a process according to any of the preceding items, wherein the (meth)acryloyl halide is a (meth)acryloyl chloride or a (meth)acryloyl bromide, preferably a (meth)acryloyl chloride.
Item 28 is a process according to any of the preceding items, wherein the (meth)acryloyl halide is acryloyl chloride or methacryloyl chloride, preferably acryloyl chloride.
Item 29 is a process according to any of the preceding items, wherein the organic solvent is selected from the group consisting of aliphatic or aromatic hydrocarbons, ethers, amides, sulfoxides and halogenated solvents, in particular chlorinated or brominated organic solvents, preferably chlorinated organic solvents.
Item 30 is a process according to any of the preceding items, wherein the organic solvent is selected from the group consisting of halogenated hydrocarbons.
Item 31 is a process according to any of the preceding items, wherein the halogenated organic solvent is dichloromethane.
Item 32 is a process according to any of the preceding items, wherein the tertiary amine comprises at least one of triisopropylamine, diisopropylethylamine, triethyl amine, trimethyl amine, methyldiethyl amine, and any mixtures thereof.
Item 33 is a process according to any of the preceding items, wherein the tertiary amine comprises diisopropylethylamine or triethyl amine, in particular diisopropylethylamine.
Item 34 is a process according to any of the preceding items, wherein the halogenating agent is selected from the group of chlorinating agents or brominating agents, preferably chlorinating agents.
Item 35 is a process according to any of the preceding items, wherein the halogenating agent is a chlorinating agent preferably selected from the group consisting of thionyl chloride, phosphoryl chloride, oxalyl chloride, and any mixtures thereof.
Item 36 is a process according to any of the preceding items, wherein the halogenating agent is a brominating agent preferably selected from the group consisting of thionyl bromide, phosphoryl bromide, and any mixtures thereof.
Item 37 is a process according to any of the preceding items, wherein the halogenating agent is selected from the group consisting of thionyl chloride, phosphoryl chloride, oxalyl chloride, thionyl bromide, phosphoryl bromide, and any mixtures thereof.
Item 38 is a process according to any of the preceding items, wherein the halogenating agent is selected from the group consisting of thionyl chloride, phosphoryl chloride, oxalyl chloride, and any mixtures thereof.
Item 39 is a process according to any of the preceding items, wherein the halogenating agent is selected from the group consisting of thionyl chloride, phosphoryl chloride, and any mixtures thereof.
Item 40 is a process according to any of the preceding items, wherein the halogenating agent is selected to be phosphoryl chloride.
Item 41 is a process according to any of the preceding items, wherein the co-agent is selected from the group consisting of linear N,N-disubstituted amides, cyclic N,N-disubstituted amides, heterocyclic N,N-disubstituted amides, and any combinations or mixtures thereof.
Item 42 is a process according to any of the preceding items, wherein the co-agent is selected from the group consisting of N,N-disubstituted heterocyclic amides and N,N-dialkyl amides, wherein the alkyl group is preferably selected from the group of methyl, ethyl, propyl and butyl.
Item 43 is a process according to any of the preceding items, wherein the co-agent is selected from the group consisting of N,N-dialkyl formamides and N,N-dialkyl acetamides, wherein the alkyl group is preferably selected from the group of methyl, ethyl, propyl and butyl.
Item 44 is a process according to any of the preceding items, wherein the co-agent is selected from the group consisting of N,N-disubstituted heterocyclic amides, in particular N-formyl morpholine.
Item 45 is a process according to any of the preceding items, wherein the co-agent is selected from the group consisting of N,N-dimethyl formamide, N,N-diethyl formamide, N,N-dimethyl acetamide, N-formyl morpholine, and any combinations or mixtures thereof.
Item 46 is a process according to any of the preceding items, wherein the co-agent is selected from the group consisting of N,N-dimethyl formamide, N,N-diethyl formamide, N-formyl morpholine, and any combinations or mixtures thereof.
Item 47 is a process according to any of the preceding items, wherein the co-agent is selected to be N,N-dimethyl formamide.
Item 48 is a process according to any of the preceding items, wherein the co-agent is different from the tertiary amine.
Item 49 is a process according to any of the preceding items, wherein the inorganic base is an alkali- or alkali earth metal hydroxide, in particular an alkali metal hydroxide preferably selected from the group consisting of sodium hydroxide, potassium hydroxide and lithium hydroxide.
Item 50 is a process according to any of the preceding items, wherein the inorganic base is sodium hydroxide or potassium hydroxide, preferably sodium hydroxide.
Item 51 is a process according to any of the preceding items, wherein the polar solvent is selected from the group consisting of water, alcohols, amides, sulfoxides, and any mixtures thereof; and wherein the polar solvent is different from the organic solvent.
Item 52 is a process according to any of the preceding items, wherein the polar solvent is water.
Item 53 is a process according to any of the preceding items, wherein the phase transfer catalyst is selected from the group consisting of salts of tertiary amines, in particular hydrogen halide salts of triethyl amine, trimethyl amine; and quaternary ammoniums salts, in particular tetraethyl ammonium halides, tetramethylammonium halides, tetraisopropylammonium halides, tetrabutylammonium halides; and any mixtures thereof.
Item 54 is a process according to any of the preceding items, wherein the phase transfer catalyst is selected from the group consisting of tetraethyl ammonium halides and tetrabutylammonium halides.
Item 55 is a process according to any of the preceding items, wherein the phase transfer catalyst is selected from the group consisting of hydrogen halide salts of triethyl amine and tetrabutylammonium halides, in particular hydrogen chloride salt of triethyl amine and tetrabutylammonium bromide.
Item 56 is a process according to any of the preceding items, wherein the molar ratio of the (meth)acrylic acid to the (meth)acryloyl halide is 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; or even 1 to at least 1.5.
Item 57 is a process according to any of the preceding items, wherein the molar ratio of the (meth)acrylic acid to the (meth)acryloyl halide is no greater than 1 to 1.5; no greater than 1 to 1.4; no greater than 1 to 1.3; or even no greater than 1 to 1.2.
Item 58 is a process according to any of the preceding items, wherein the molar ratio of the (meth)acrylic acid to the (meth)acryloyl halide is in a range between 1 to 0.8 and 1 to 1.5, between 1 to 1 and 1 to 1.5, between 1 to 1 and 1 to 1.4, between 1 to 1 and 1 to 1.3, or even between 1 to 1 and 1 to 1.2.
Item 59 is a process according to any of the preceding items, wherein the molar ratio of the (meth)acrylic acid to the (meth)acryloyl halide is about 1 to 1 or 1 to 1.1.
Item 60 is a process according to any of the preceding items, wherein the molar ratio of the (meth)acrylic acid to the tertiary amine is 1 to at least 0.8; 1 to at least 0.9; 1 to at least 1; 1 to at least 1.02; 1 to at least 1.05; 1 to at least 1.1; 1 to at least 1.15; 1 to at least 1.2; 1 to at least 1.3; 1 to at least 1.4; 1 to at least 1.5; 1 to at least 2; 1 to at least 2.5; or even 1 to at least 3.
Item 61 is a process according to any of the preceding items, wherein the molar ratio of the (meth)acrylic acid to the tertiary amine is no greater than 1 to 3; no greater than 1 to 2.5; no greater than 1 to 2; no greater than 1 to 1.5; no greater than 1 to 1.4; no greater than 1 to 1.3; or even no greater than 1 to 1.2.
Item 62 is a process according to any of the preceding items, wherein the molar ratio of the (meth)acrylic acid to the tertiary amine is in a range between 1 to 0.8 and 1 to 3, between 1 to 0.8 and 1 to 2.5, between 1 to 0.8 and 1 to 2, between 1 to 0.8 and 1 to 1.5, between 1 to 1 and 1 to 1.5, between 1 to 1 and 1 to 1.4, between 1 to 1 and 1 to 1.3, or even between 1 to 1 and 1 to 1.2.
Item 63 is a process according to any of the preceding items, wherein the molar ratio of the (meth)acrylic acid to the tertiary amine is about 1 to 1 or 1 to 1.5.
Item 64 is a process according to any of the preceding items, wherein the molar ratio of the halogenating agent to the (meth)acrylic acid is 1 to at least 1.5; 1 to at least 1.6; 1 to at least 1.7; 1 to at least 1.8; 1 to at least 1.9; 1 to at least 2.0; 1 to at least 2.1; or even 1 to at least 2.2.
Item 65 is a process according to any of the preceding items, wherein the molar ratio of the halogenating agent to the (meth)acrylic acid is no greater than 1 to 2.5; no greater than 1 to 2.4; no greater than 1 to 2.2; or even no greater than 1 to 2.0.
Item 66 is a process according to any of the preceding items, wherein the molar ratio of the halogenating agent to the (meth)acrylic acid is in a range between 1 to 1.5 and 1 to 2.5, between 1 to 1.6 and 1 to 2.4, between 1 to 1.7 and 1 to 2.2, between 1 to 1.8 and 1 to 2.2, between 1 to 1.9 and 1 to 2.2, or even between 1 to 1.9 and 1 to 2.1.
Item 67 is a process according to any of the preceding items, wherein the molar ratio of the halogenating agent to the (meth)acrylic acid is about 1 to 2.
Item 68 is a process according to any of the preceding items, wherein the molar ratio of the halogenating agent to the co-agent is 1 to at least 1; 1 to at least 1.2; 1 to at least 1.4; or even 1 to at least 1.5.
Item 69 is a process according to any of the preceding items, wherein the molar ratio of the halogenating agent to the co-agent is no greater than 1 to 2.5; no greater than 1 to 2.4; no greater than 1 to 2.2; no greater than 1 to 2; no greater than 1 to 1.8; no greater than 1 to 1.6; even no greater than 1 to 1.5.
Item 70 is a process according to any of the preceding items, wherein the molar ratio of the halogenating agent to the co-agent is in a range between 1 to 1 and 1 to 2.5, between 1 to 1 and 1 to 2.2, between 1 to 1.2 and 1 to 2, between 1 to 1.3 and 1 to 1.8, between 1 to 1.3 and 1 to 1.8, or even between 1 to 1.4 and 1 to 1.6.
Item 71 is a process according to any of the preceding items, wherein the molar ratio of the halogenating agent to the co-agent is about 1 to 1.5.
Item 72 is a process according to any of the preceding items, wherein the amount of the phase transfer catalyst is in a range of from 5 to 50 mol %, from 5 to 45 mol %, from 7 to 45 mol %, from 7 to 40 mol %, from 7 to 35 mol %, from 7 to 30 mol %, or even from 10 to 30 mol %, based on the molar equivalent of the (meth)acrylic acid.
Item 73 is a process according to any of the preceding items, wherein the molar ratio of the (meth)acrylic acid to the inorganic base is in a range between 1 to 0.8 and 1 to 1.1, or even between 1 to 1 and 1 to 1.05.
Item 74 is a process according to any of the preceding items, wherein the reaction product stream comprises the (meth)acrylic anhydride in an amount of at least 80 wt %, at least 85 wt %, at least 90 wt %, at least 95 wt %, or even at least 98 wt % based on the total weight of the (meth)acrylic anhydride, the (meth)acrylic acid, and the organic by-products in the reaction product stream.
Item 75 is a process according to any of the preceding items, wherein the conversion rate of the (meth)acrylic acid into the (meth)acrylic anhydride is of at least 80 mol %, at least 85 mol %, at least 90 mol %, at least 95 mol %, or even at least 98 mol % based on the molar equivalent of the (meth)acrylic acid or (meth)acrylic acid salt, and when determined by ¹H NMR spectroscopy.
Item 76 is a process according to any of the preceding items, wherein the reactants comprise polymerization inhibitors, in particular polymerization inhibitors selected from the group of phenothiazines and hydroquinones, in particular hydroquinone monomethyl ethers and hydroquinone methyl esters.
Item 77 is a process according to any of the preceding items, which does not comprise a transanhydrification step.
Item 78 is a process according to any of the preceding items, whereby substantially no gaseous by-products are formed in the reaction chamber of the flow reactor(s), in particular no gaseous by-products selected from the group of hydrochloric acid, carbon dioxide, carbon monoxide and sulphur dioxide.
Item 79 is a process according to any of the preceding items, which is a continuous process.
Item 80 is the use of a polar solvent for the manufacturing of a (meth)acrylic anhydride in a flow reactor.
Item 81 is the use according to item 80, wherein the polar solvent is or comprises water.

### EXAMPLES

The present disclosure is further illustrated by the following examples. These examples are merely for illustrative purposes only and are not meant to be limiting on the scope of the appended claims.

The following abbreviations are used in this section: NMR=nuclear magnetic resonance, ml=milliliters, min=minutes, mm=millimeters, ppm=parts per million, mol % = mole percent. Abbreviations of materials used in this section, as well as descriptions of the materials, are provided in Table 1.

**Table 1**

| **Material** | **Description** |
|---|---|
| AA | acrylic acid, available from Aldrich, Belgium |
| AC | acryloyl chloride, obtained in the examples |
| AZ | acrylic anhydride, obtained in the examples |
| MA | methacrylic acid, available from Aldrich, Belgium |
| MC | methacryloyl chloride, obtained in the examples |
| MAZ | methacrylic anhydride, obtained in the examples |
| ACL | (meth)acryloyl chloride, obtained or used in the examples |
| ANZ | (meth)acrylic anhydride, obtained in the examples |
| NA | sodium acrylate, obtained in the examples |
| NMA | sodium methacrylate, obtained in the examples |
| ANS | sodium (meth)acrylate, obtained in the examples |
| PTC1 | phase transfer catalyst 1: Et₃N.HCl, available from Aldrich, Belgium |
| PTC2 | phase transfer catalyst 1: Bu₄N-Br, available from Aldrich, Belgium |
| DIPEA | diisopropylethylamine, available from Aldrich, Belgium |
| DMF | N,N-dimethyl formamide, available from Aldrich, Belgium |
| DEF | N,N-diethyl formamide, available from Aldrich, Belgium |
| NFM | N-formyl morpholine, available from BASF, Belgium |
| POCl₃ | phosphoryl chloride, available from Aldrich, Belgium |
| DCM | dichloromethane, available from Aldrich, Belgium |

### Test methods and characterization:

### Molar ratio

The term "Molar ratio" is used throughout this section to mean the ratio or ratios of indicated reactants incorporated into the reaction chamber of the flow reactor.

### Conversion Rate

The term "Conversion" is used throughout this section to mean the molar percentage of the (meth)acrylic acid which is actually converted into the corresponding (meth)acrylic anhydride The conversion rate is determined by ¹H NMR spectroscopy on the unpurified reaction mixture, as described below, under "Characterization."

### Characterization

*NMR*: Analysis by NMR is made using a Bruker Avance 300 Digital NMR spectrometer equipped with Bruker 5 mm BBFO 300 MHz Z-gradient high resolution-ATM probe. The samples are placed in NMR tubes available under the trade designation "WG-5M-ECONOMY" from Aldrich, Belgium. TMS (tetramethylsilane, available from Aldrich, Belgium) is added as a zero ppm reference. Proton NMR spectra are acquired using the following parameters:
Pulse Angle: 30°
Number of Scans: 128
Acquisition Time: 5.3 s
Relaxation time: 2.0 s

Except where noted, NMR confirmed the identity of the desired products.

### Equipment employed:

The experiments and reactions are performed using a flow reactor built of PFA-tubing having an inner diameter of 0.50 mm available under the trade designation "IDEX 1512L" from Achrom, Belgium. The flow reactor is a tube reactor having a circular circuitous tube shape, an inner diameter of about 0.50 mm and a total volume of 0.5 ml. The flow reactor is suitably connected to syringe pumps commercially available under the trade designation Fusion Touch or Fusion Classic from Chemtrix BV, delivering at least two reactant streams from at least two gas-tight syringes, available under the trade designation "Hamilton Syringe 10 ml 1000 series GASTIGHT" from Hamilton, through PFA tubing with an inner diameter of 1.0 mm, available under the trade designation "IDEX 1507" from Achrom, Belgium, to the reaction chamber of the flow reactor. The gas-tight syringes are connected to the system using an ETFE luer lock (available under the trade designation "IDEX P-628" from Achrom, Belgium) and are mixed together in a ETFE T-connector having a diameter of 0.5 mm (available under the trade designation "IDEX P-632" from Achrom, Belgium). The flow reactor is provided with at least one addition port. The at least two reactant addition streams are incorporated into the reaction chamber of the flow reactor, where a reaction product stream is formed. The reaction product stream exits the flow reactor through a product port and flows through PFA tubing with an inner diameter of 1 mm, connected to the product port using connectors available from Achrom, Belgium, into a collection vessel. In some other examples, the reaction product stream directly exits the flow reactor through the product port. The flow reactor is placed at 20°C or heated at the appropriate temperature in an oil bath.

### Examples:

### Examples 1 to 2 and Comparative Example 1:

For Ex.1 to Ex.2 and comparative example CE-1, the following general procedure is carried out using two flow reactors as described above at 20°C. A solution of tertiary amine DIPEA in organic solvent DCM is prepared as a first addition stream (Stream I) and incorporated into a first flow reactor through a first syringe at a flow speed of 0.12 ml/min. Comparative example CE-1 does not comprise an organic solvent. Pure (meth)acrylic acid is incorporated into the first flow reactor as a second addition stream (Stream II) through a second syringe at a flow speed of 0.028 ml/min. The resulting intermediate reaction product stream is left to react for 2 minutes at 20°C and then incorporated as a third addition stream (Stream III) into a second flow reactor through a third syringe at a combined flow speed of 0.15 ml/min. Pure (meth)acryloyl chloride is incorporated as a fourth addition stream (Stream IV) into the second flow reactor through a fourth syringe at a flow speed of 0.030 ml/min. The molar ratios of [(meth)acrylic acid : tertiary amine : (meth)acrylol chloride), referred to below as (acid : DIPEA : ACL), incorporated into the reaction chamber(s), the residence time (RT in min) in the second flow reactor as well as the conversion rate, determined by ¹H NMR spectroscopy, are specified in Table 2.

**Table 2**

| Example | Stream II | Stream IV | Molar ratios (acid:DIPEA:ACL) | RT (min) | ANZ | Conversion (mol%) |
|---|---|---|---|---|---|---|
| **Ex.1** | AA | AC | 1:1:1 | 1.7 | AZ | 100 |
| **Ex.2** | MA | MAC | 1:1:1 | 1.6 | MAZ | 100 |
| **CE-1** | AA | AC | 1:2:1 | 3 | - | 0^{(*)} |

| | | | | | | |
|---|---|---|---|---|---|---|
| (*) due to clogging of the reactor. | | | | | | |

As can be seen from the results, example CE-1 not comprising an organic solvent leads to irreversible clogging of the flow reactor.

### Examples 3 to 8:

For Ex.3 to Ex.8, the following general procedure is carried out using two flow reactors as described above at 20°C. A blend of (meth)acrylic acid and co-agent is prepared as a first addition stream (Stream I) and incorporated into a first flow reactor through a first syringe at a flow speed of about 0.13 ml/min. Pure POCl₃ is incorporated into the first flow reactor as a second addition stream (Stream II) through a second syringe at a flow speed of about 0.045 ml/min. The resulting intermediate reaction product stream is left to react for the time specified below (RT1) at 20°C and then incorporated as a third addition stream (Stream III) into a second flow reactor through a third syringe at a combined flow speed of about 0.18 ml/min. The resulting intermediate product comprises a mixture of approximately 1 molar equivalent of (meth)acrylic acid and 1 molar equivalent of (meth)acryloyl chloride. A solution of tertiary amine DIPEA in organic solvent DCM is incorporated as a fourth addition stream (Stream IV) into the second flow reactor through a fourth syringe at a flow speed of about 0.65 ml/min. The molar ratios of [(meth)acrylic acid : POCl₃ : co-agent: DIPEA] incorporated into the reaction chamber(s) are 2 : 1 : 1.5 : 3. The residence time in the first (RT1 in min) and the second flow reactor (RT2 in min), as well as the conversion rate, determined by ¹H NMR spectroscopy, are specified in Table 3.

**Table 3**

| Example | Stream I | | RT1 (min) | RT2 (min) | ANZ | Conversion (mol%) |
|---|---|---|---|---|---|---|
| | Acid | Co-agent | | | | |
| **Ex.3** | AA | DMF | 1 | 2.6 | AZ | 100 |
| **Ex.4** | MA | DMF | 1 | 2.75 | MAZ | 100 |
| **Ex.5** | AA | NFM | 5 | 1.4 | AZ | 100 |
| **Ex.6** | MA | NFM | 5 | 1.5 | MAZ | 100 |
| **Ex.7** | AA | DEF | 1 | 2.85 | AZ | 100 |
| **Ex.8** | MA | DEF | 1 | 3 | MAZ | 100 |

### Examples 9 to 11:

For Ex.9 to Ex.11, the following general procedure is carried out using the flow reactor as described above at the specified temperature. A solution of (meth)acrylic acid, sodium hydroxide (as inorganic base) and a phase transfer catalyst (PTC) in water is prepared as a first addition stream (Stream I) and incorporated into the flow reactor through a first syringe at a flow speed of about 0.125 ml/min. The first addition stream comprises sodium (meth)acrylate (referred to below as NA or NMA). A solution of (meth)acryloyl chloride in organic solvent DCM is prepared as a second addition stream (Stream II) and incorporated into the flow reactor through a second syringe at a flow speed of about 0.073 ml/min. The molar ratios of [sodium (meth)acrylate : (meth)acryloyl chloride], referred to below as (ANS : ACL), and amount of PTC (in grams) incorporated into the reaction chamber, the residence time (RT in min), the temperature of the reaction chamber (in °C) as well as the conversion rate, determined by ¹H NMR spectroscopy, are specified in Table 4.

**Table 4**

| Example | Stream I | | Molar ratios (ANS:ACL) | PTC (grams) | T (°C) | RT (min) | ANZ | Conversion (mol%) |
|---|---|---|---|---|---|---|---|---|
| **Ex.9** | NA | PTC1 | 1:1.3 | 3 | 40 | 15 | AZ | 85 |
| **Ex.10** | NA | PTC2 | 1:1 | 1 | 20 | 10 | AZ | 83 |
| **Ex.11** | NMA | PTC2 | 1:1 | 1 | 20 | 10 | MAZ | 100 |

As can be seen from the results, high conversion of (meth)acrylic acid into (meth)acrylic anhydride is obtained in a two-phase system comprising water and dichloromethane. This is surprising finding since (meth)acryloyl chlorides as well as (meth)acrylic anhydrides are highly water sensitive.

## Claims

1. A process for the manufacturing of a (meth)acrylic anhydride, wherein the process comprises the steps of:
A. providing a flow reactor comprising a reaction chamber;
B. providing reactants and reagents comprising:
a) a (meth)acryloyl halide;
b) an organic solvent;
c) (meth)acrylic acid;
d) and either:
i. a tertiary amine; or
ii. an inorganic base and a polar solvent; and
C. incorporating the reactants and reagents into the reaction chamber of the flow reactor, thereby forming a reaction product stream comprising the (meth)acrylic anhydride.

2. A process according to claim 1, wherein the reactants and reagents comprise:
a) a (meth)acryloyl halide;
b) an organic solvent;
c) (meth)acrylic acid; and
d) a tertiary amine.

3. A process according to claim 2, wherein the (meth)acryloyl halide is provided by reacting the (meth)acrylic acid with a halogenating agent and a co-agent in the reaction chamber of a secondary flow reactor thereby forming the (meth)acryloyl halide for use in the process according to any of claim 1 or 2.

4. A process according to claim 1, wherein the reactants and reagents comprise:
a) a (meth)acryloyl halide;
b) an organic solvent;
c) (meth)acrylic acid;
d) an inorganic base;
e) a polar solvent; and
f) optionally, a phase transfer catalyst.

5. A process according to any of the preceding claims, wherein the (meth)acryloyl halide is a (meth)acryloyl chloride or a (meth)acryloyl bromide, preferably a (meth)acryloyl chloride.

6. A process according to any of the preceding claims, wherein the (meth)acryloyl halide is acryloyl chloride or methacryloyl chloride, preferably acryloyl chloride.

7. A process according to any of the preceding claims, wherein the organic solvent is selected from the group consisting of aliphatic or aromatic hydrocarbons, ethers, amides, sulfoxides and halogenated solvents, in particular chlorinated or brominated organic solvents, preferably chlorinated organic solvents.

8. A process according to any of the preceding claims, wherein the tertiary amine comprises at least one of triisopropylamine, diisopropylethylamine, triethyl amine, trimethyl amine, methyldiethyl amine, any mixtures thereof.

9. A process according to any of the preceding claims, wherein the halogenating agent is selected from the group of chlorinating agents or brominating agents, preferably chlorinating agents.

10. A process according to any of the preceding claims, wherein the halogenating agent is a chlorinating agent preferably selected from the group consisting of thionyl chloride, phosphoryl chloride, oxalyl chloride, and any mixtures thereof.

11. A process according to any of the preceding claims, wherein the co-agent is selected from the group consisting of linear N,N-disubstituted amides, cyclic N,N-disubstituted amides, heterocyclic N,N-disubstituted amides, and any combinations or mixtures thereof.

12. A process according to any of the preceding claims, wherein the inorganic base is an alkali- or alkali earth metal hydroxide, in particular an alkali metal hydroxide preferably selected from the group consisting of sodium hydroxide, potassium hydroxide and lithium hydroxide.

13. A process according to any of the preceding claims, wherein the polar solvent is selected from the group consisting of water, alcohols, amides, sulfoxides, and any mixtures thereof; and wherein the polar solvent is different from the organic solvent.

14. A process according to any of the preceding claims, wherein the phase transfer catalyst is selected from the group consisting of salts of tertiary amines, in particular salts of triethyl amine, trimethyl amine; and quaternary ammoniums salts, in particular tetraethyl ammonium halides, tetramethylammonium halides, tetraisopropylammonium halides, tetrabutylammonium halides; and any mixtures thereof.

15. Use of a polar solvent for the manufacturing of a (meth)acrylic anhydride in a flow reactor.
